(19) Europäisches Patentamt
European Patent Office
Office européen des brevets

(11) Veröffentlichungsnummer: **0 447 965 A1**

(12) # EUROPÄISCHE PATENTANMELDUNG

(21) Anmeldenummer: **91103926.1**

(51) Int. Cl.5: **C07D 263/56**

(22) Anmeldetag: **14.03.91**

(30) Priorität: **21.03.90 DE 4009027**

(43) Veröffentlichungstag der Anmeldung:
**25.09.91 Patentblatt 91/39**

(84) Benannte Vertragsstaaten:
**AT BE CH DE DK ES FR GB GR IT LI LU NL SE**

(71) Anmelder: **BASF Aktiengesellschaft**
**Carl-Bosch-Strasse 38**
**W-6700 Ludwigshafen(DE)**

(72) Erfinder: **Gaeng, Manfred, Dr.**
**An der Tuchbleiche 11**
**W-6712 Bobenheim-Roxheim(DE)**
Erfinder: **Saukel, Heinz, Dr.**
**Im Rosengarten Ost 15**
**W-6701 Friedelsheim(DE)**
Erfinder: **Hoffmann, Wolfgang, Dr.**
**Friedrich-Schanzlin-Strasse 13**
**W-6710 Frankenthal(DE)**
Erfinder: **Koenig, Josef**
**In der Lache 21**
**W-6719 Zellertal(DE)**

(54) **Verfahren zur Herstellung von 2-Methylbenzoxazol.**

(57) Verfahren zur Herstellung von 2-Methylbenzoxazol durch Zyklisierung von Acetophenol in Gegenwart des Hilfsstoffes Pluriol 400 E bei gleichzeitiger Wasserabspaltung, wobei die Trocknung und Aufschmelzung des Aceotophenols, die Zyklisierung des Acetophenols und die Abdestillierung des Reaktionswassers und des 2-Methylbenzoxazols aus dem Reaktionsgemisch nach der Zyklisierung in einem Trockner erfolgt.

EP 0 447 965 A1

Die Erfindung betrifft ein Verfahren zur Herstellung von 2-Methylbenzoxazol durch Zyklisierung von Acetophenol in Gegenwart des Hilfsstoffs Pluriol 400 E bei gleichzeitiger Wasserabspaltung.

Die Reaktion verläuft nach folgender Gleichung:

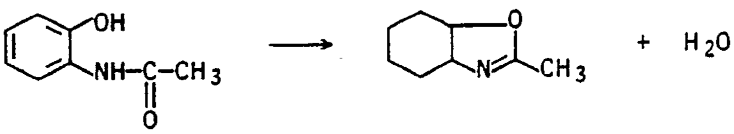

Die Herstellung von 2-Methylbenzoxazol erfolgt nach dem Stand der Technik wie nachstehend:
2400 kg Acetophenol + (3000 kg $H_2O$ + 230 kg Pluriol 400 E) → 1720 kg 2-Methylbenzoxazol + 394 kg Rückstand + 286 kg $H_2O$ + (3000 kg $H_2O$ + 230 kg Pluriol 400 E)
Dies entspricht einem Umsatz von Acetophenol zu 2-Methylbenzoxazol von ca. 72 %.

Hierbei erfolgt die Reaktion in einem Rührbehälter bei ca. 160-170 °C und nachfolgendem Abdestillieren von Wasser und 2-Methylbenzoxazol aus dem Reaktionsgemisch. Acetophenol wird hierbei als getrocknetes Produkt der Reaktion zugeführt.

Als Nachteil des Verfahrens erweist sich hierbei - wie aus der Mengenbilanz ersichtlich - daß der Umsatz nur ca. 72 % beträgt, gleichzeitig Rückstand und Pluriol 400 E zusammen mit Wasser der Entsorgung zugeführt werden müssen und das Trocknen des Acetophenols in einer gesonderten Apparatur durchgeführt werden muß.

Es stellt sich daher die Aufgabe ein Verfahren zur Herstellung von 2-Methylbenzoxazol zu konzipieren, wobei der Umsatz von Acetophenol zu 2-Methylbenzoxazol verbessert wird, d.h. die Ausbeute des Verfahrens erhöht wird, der Einsatz des Hilfsstoffes Puriol 400 E bei der Reaktion entfallen kann, und damit die Entsorgung vermindert werden kann und letztendlich für die Trocknung des Acetophenols keine gesonderte Apparatur benötigt wird.

Letzteres bedeutet, das Acetophenol in dem Zustand - nämlich feucht - wie es aus der vorhergehenden Verfahrensstufe anfällt, der Zyklisierung zuzuführen.

Diese Aufgabe wird erfindungsgemäß dadurch gelöst, daß die Trocknung und Aufschmelzung des Acetophenols, die Zyklisierung des Acetophenols und die Abdestillierung des Reaktionswassers und des 2-Methylbenzoxazols aus dem Reaktionsgemisch nach der Zyklisierung in einem Trockner erfolgt.

Weitere Merkmale des Verfahrens sind Gegenstand der Unteransprüche.

Ein Ausführungsbeispiel des erfindungsgemäßen Verfahrens mit den wesentlichen Merkmalen ist in der Zeichnung dargestellt und wird im folgenden näher beschrieben.

Die Zeichnung zeigt ein vereinfachtes Verfahrensschema.

Acetophenol 1 - ein wasserfeuchter Filterkuchen, wie er aus der vorhergehenden Verfahrensstufe anfällt - wird einem Trockner 2, beispielsweise einem handelsüblichen Schaufeltrockner zugeführt. Dieser Schaufeltrockner zeichnet sich insbesondere durch eine hohe volumenbezogene Wärmeaustauschfläche aus. In dem Schaufeltrockner werden durch Wärmeübertragung mittels eines Wärmeträgers 3 nacheinander folgende Verfahrensmaßnahmen durchgeführt:

- Trocknen des wasserfeuchten Filterkuchens,
- Aufschmelzen des getrockneten Filterkuchens und gleichzeitiges Zyklisieren des Acetophenols zu 2-Methylbenzoxazol unter Abspaltung von Reaktionswasser,
- Abdestillieren des Reaktionswassers 4 während des Zyklisierens, beginnend bei Normaldruck, im Laufe des Zyklisierens dann unter Vakuum zur Reduzierung der Rückstandsbildung, und nach Abdestillieren des Reaktionswassers,
- Abdestillieren des 2-Methylbenzoxazols 5 und anschließend
- Entfernen des Rückstandes 6 aus dem Schaufeltrockner.

Mittels des aufgezeigten Verfahrens konnten eindeutige Vorteile gegenüber dem Verfahren nach dem Stand der Technik erzielt werden, nämlich

- Ausbeutesteigerung von ca. 72 % auf ca. 78-80 %,
- Verzicht auf den Hilfsstoff Puriol 400 E,
- Verminderung des Anfalls von Produkten, die der Entsorgung zugeführt werden müssen,
- Verringerung der Investitionskosten bei Konzipierung einer neuen Anlage, und
- Wegfall des Handlings von getrocknetem Acetophenol, was aus arbeitshygienischen Gründen erstrebenswert war.

**Patentansprüche**

1. Verfahren zur Herstellung von 2-Methylbenzoxazol durch Zyklisierung von Acetophenol in Gegenwart des Hilfsstoffes Pluriol 400 E bei gleichzeitiger Wasserabspaltung, dadurch gekennzeichnet, daß die Trocknung und Aufschmelzung des Acetophenols, die Zyklisierung des Acetophenols und die Abdestillierung des Reaktionswassers und des 2-Methylbenzoxazols aus dem Reaktionsgemisch nach der Zyklisierung in einem Trockner erfolgt.

2. Verfahren nach Anspruch 1, dadurch gekennzeichnet, daß als Trockner ein Schaufeltrockner oder Discotherm-Trockner verwendet wird.

3. Verfahren nach den Ansprüchen 1 und 2, dadurch gekennzeichnet, daß der Hilfsstoff Pluriol 400 E entfällt.

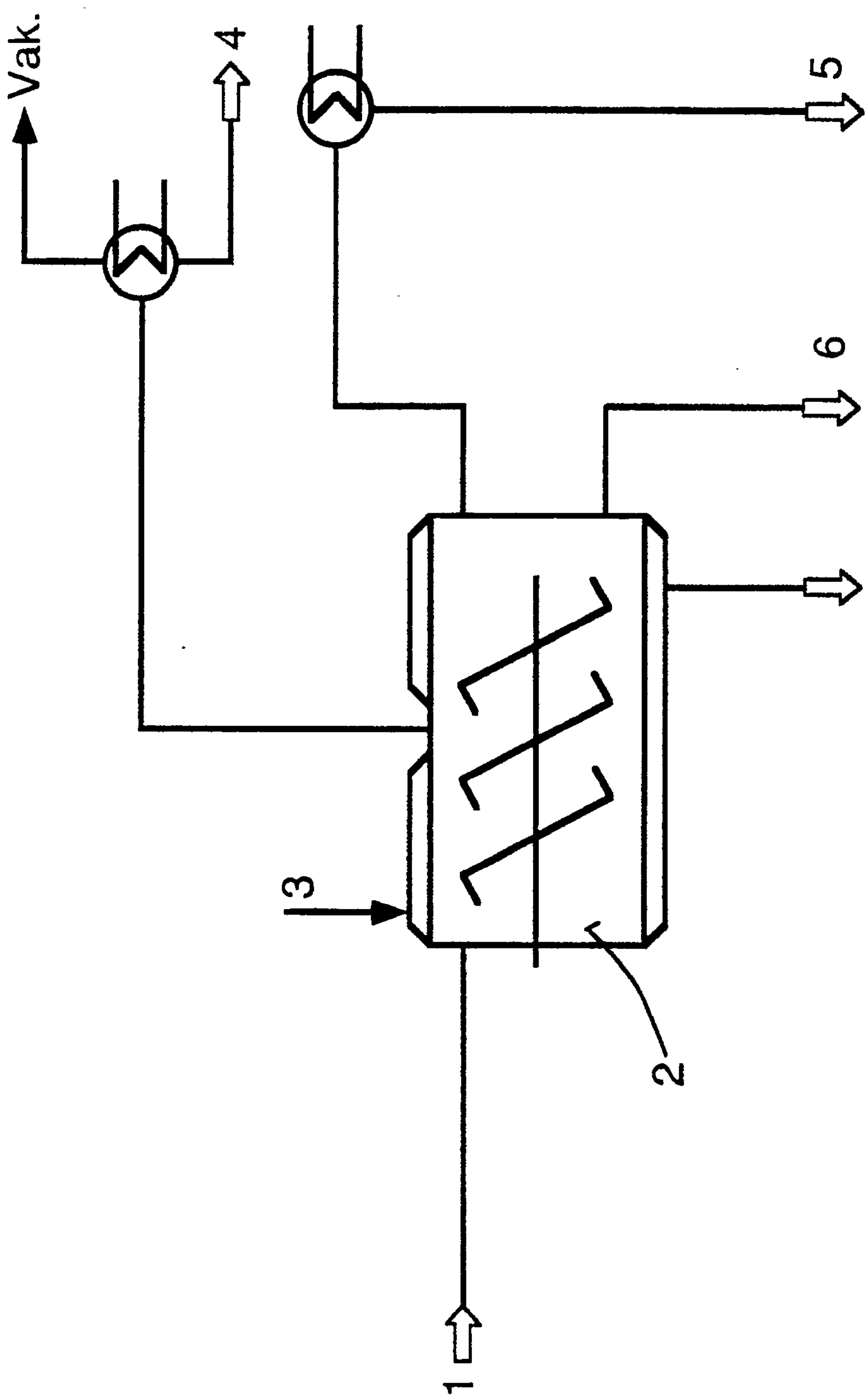
Vak.
4
5
6
3
2
1

Europäisches Patentamt

# EUROPÄISCHER RECHERCHENBERICHT

Nummer der Anmeldung

**EP 91 10 3926**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS, Band 32, Nr. 4, 20. Februar 1938, Seiten 1263-1264, Zusammenfassung Nr. 1263, 7-9, Columbus, Ohio, US; B. BEILENSON: "A preparation of 1-methylbenzoxazole", & J. SOC. CHEM. IND. 56, 302T(1937) * Zusammenfassung * | 1 | C 07 D 263/56 |
| A | EP-A-0 006 195 (HOECHST AG) * Beispiele 8,9 * | 1 | |

| RECHERCHIERTE SACHGEBIETE (Int. Cl.5) |
|---|
| C 07 D 263/00 |

Der vorliegende Recherchenbericht wurde für alle Patentansprüche erstellt

| Recherchenort | Abschlußdatum der Recherche | Prüfer |
|---|---|---|
| Den Haag | 24 Mai 91 | HENRY J.C. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument